# EUROPEAN PATENT APPLICATION

(11) **EP 3 009 194 A1**
(43) Date of publication of application: **20.04.2016**
(21) Application number: 15180711.2
(22) Date of filing: 12.08.2015
(51) Int. Cl.: B05B 1/34, B05C 17/005, A61B 17/00

(54) **APPLICATOR, MIXING TIP AND METHOD FOR DISPENSING A MIXED FLUID**

(30) Priority: 20.08.2014 US 201462039627 P; 29.07.2015 US 201514812019
(71) Applicant: NORDSON CORPORATION, Westlake, OH 44145 (US)
(72) Inventor: KIRK, Thomas A, Hastings, MN Minnesota 55033 (US)
(74) Representative: Findlay, Alice Rosemary

(57) **Abstract**

An applicator for dispensing a fluid for use during a medical procedure includes a dispensing body for containing the fluid. The dispensing body includes a conduit, and a mixing tip operatively connected to the conduit for receiving the fluid from the dispensing body. The mixing tip includes a tip body having an interior passage. The interior passage includes an inlet at a first end fluidly connected to the conduit for receiving the fluid from the conduit and an outlet in a distal wall at a second end opposite to the first end. An insert body is positioned within the interior passage. A first passage is positioned between the insert body and the tip body and fluidly couples the interior passage with the outlet in the distal wall. A mixing chamber is positioned between the insert body and the distal wall. The mixing chamber includes a static mixing element.

## Description

The present invention relates generally to an applicator and method for dispensing a fluid and, more particularly, dispensing a mixed fluid topically during a medical procedure.

Generally, it is well-known to dispense liquids in the form of "sprayed" droplets, such as biomaterials for use in surgical procedures. More specifically, a plurality of reactive liquids may or may not be sprayed under the influence of pressurized gas to disperse and dispense the droplets on the human body or within the human body to beneficially affect the outcome of the surgical procedure. For instance, two highly reactive fluids may be sprayed onto an anatomical site for reducing the flow of blood by hemostatic clotting or creating tissue barriers to prevent anatomical tissues from adhering together during and/or after the surgical procedure. Ideally, these reactive liquids are isolated prior to being discharged from the instrument. As such, the reactive liquids mix and react during discharge from the instrument for application at the anatomical site. Thus, the beneficial characteristics of the reacting fluids are preserved until needed for use.

There is a continuing need for improvements in this area, including the provision of devices and methods that result in uniform and balanced mixing, and optimal discharge characteristics while maintaining cost efficiencies.

In one general aspect, the invention provides an applicator for dispensing a fluid for use during a medical procedure. The applicator includes a dispensing body for containing the fluid and including a conduit. The applicator further includes a mixing tip operatively connected to the conduit for receiving the fluid from the dispensing body. The mixing tip includes a tip body having an interior passage extending along a length. The interior passage includes an inlet at a first end fluidly connected to the conduit for receiving the fluid from the conduit, and an outlet in a distal wall at a second end opposite to the first end. The mixing tip further comprises an insert body, a first passage and a mixing chamber. The insert body is positioned within the interior passage. The first passage is positioned between the insert body and the tip body and fluidly couples the interior passage with the outlet in the distal wall. The mixing chamber is positioned between the insert body and the distal wall and includes a static mixing element.

In another aspect the invention provides a mixing tip for discharging a fluid from a dispensing body for use during a medical procedure. The mixing tip includes a tip body, an insert body and a mixing chamber. The tip body includes an interior passage extending along a length. The interior passage includes an inlet at a first end configured for fluidly connecting the dispensing body for receiving the fluid from the dispensing body and an outlet in a distal wall at a second end opposite to the first end. The insert body is positioned within the interior passage. A first passage is positioned between the insert body and the tip body and fluidly couples the interior passage with the outlet in the distal wall. The mixing chamber is positioned between the insert body and the distal wall and includes a static mixing element.

In another aspect the invention provides a method of dispensing a mixed fluid during a medical procedure with a mixing tip, the mixing tip having an insert body positioned within a tip body. The method includes introducing a first fluid and a second fluid into an interior passage of the tip body. The first and second fluids are directed through a passage between the tip body and the insert body. The first and second fluids are directed from the passage into a mixing chamber located between the insert body and a distal wall of the tip body. The first and second fluids are mixed within the mixing chamber and then discharged from the mixing tip.

Various additional features and advantages of the invention will become more readily apparent during the following detailed description taken in conjunction with the drawings herein.

The invention will now be further described by way of example with reference to the accompanying drawings, in which:
FIG. 1 is a perspective view of an embodiment of an applicator having a mixing tip for dispensing a fluid in accordance with the invention.
FIG. 2A is an exploded front perspective view of the mixing tip of FIG. 1.
FIG. 2B is an exploded rear perspective view of the mixing tip of FIG. 1.
FIG. 3 is a cross-sectional view taken along a central axis of the mixing tip of FIG. 1.
FIG. 4 is a cross-sectional view taken along section line 4-4 of FIG. 3 showing a distal wall of the mixing tip of FIG. 1.
FIG. 5 is similar to FIG. 4, but shows a perspective view of the distal wall.

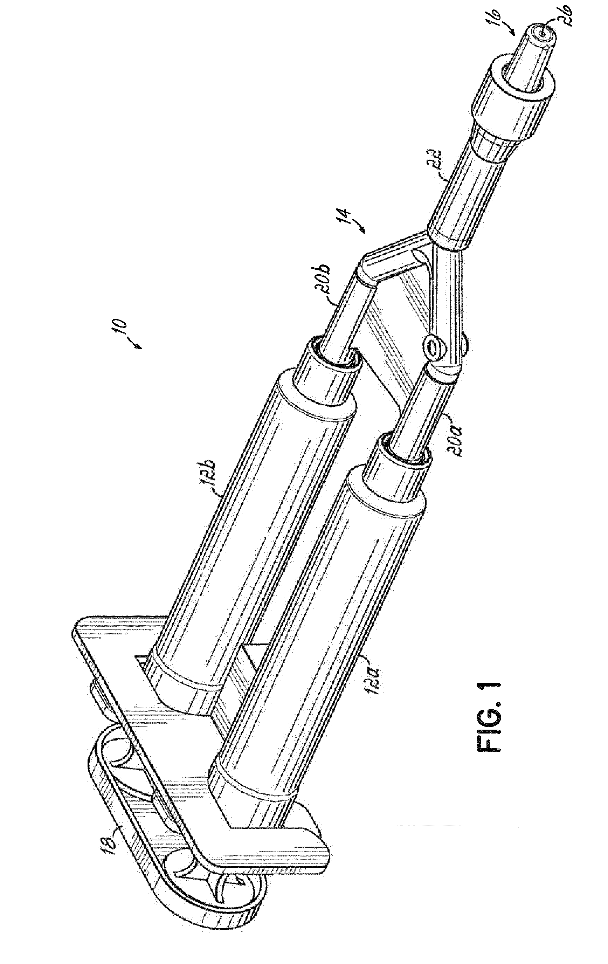

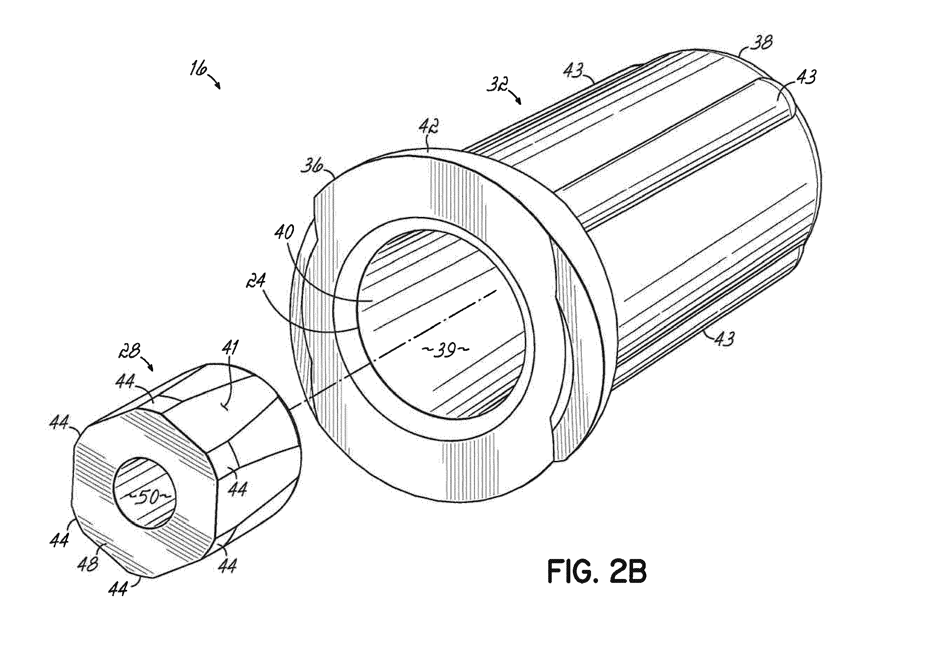

FIG. 1 illustrates an applicator 10 having first and second syringes 12a, 12b, a connector 14, and a mixing tip 16 for dispensing first and second fluids as a mixed fluid. The first and second syringes 12a, 12b respectively contain the first and second fluids that are compressed via a plunger 18 toward the distally connected connector 14. The connector 14 defines a first conduit 20a fluidly connected to the first syringe 12a and a second conduit 20b fluidly connected to the second syringe 12b. The connector 14 further defines a third conduit 22 fluidly connected to both the first and second conduits 20a, 20b for simultaneously receiving the first and second fluids therein. The third conduit 22 fluidly connects to the mixing tip 16 such that the mixing tip 16 receives the first and second fluids through a proximal inlet 24 (see FIG. 2B), mixes the first and second fluids into the mixed fluid, and discharges the mixed fluid from a distal outlet 26 of the mixing tip 16. Generally, the first and second syringes 12a, 12b may be any medical instrument having a dispensing body for dispensing one or more fluids. More particularly, the first and second fluids are each reactive liquids that may be dispensed as droplets from the mixing tip 16 during medical procedures. For example, the first and second reactive liquids may be blood and thrombin for encouraging blood coagulation. Such medical procedures for use with the applicator 10 may include topical applications and open surgical applications. With respect to the use of the terms "distal" and "proximal," it will be appreciated that such directions and/or locations are intended to describe relative locations longitudinally along exemplary embodiments of the applicator 10. Similarly, a generally longitudinal direction extends along a length of the applicator 10 in either a distal or proximal direction. And a transverse direction extends generally orthogonal to or across the longitudinal direction at any angle. It is not intended that these terms or any other spatial references limit the invention to any of the exemplary embodiments described herein.

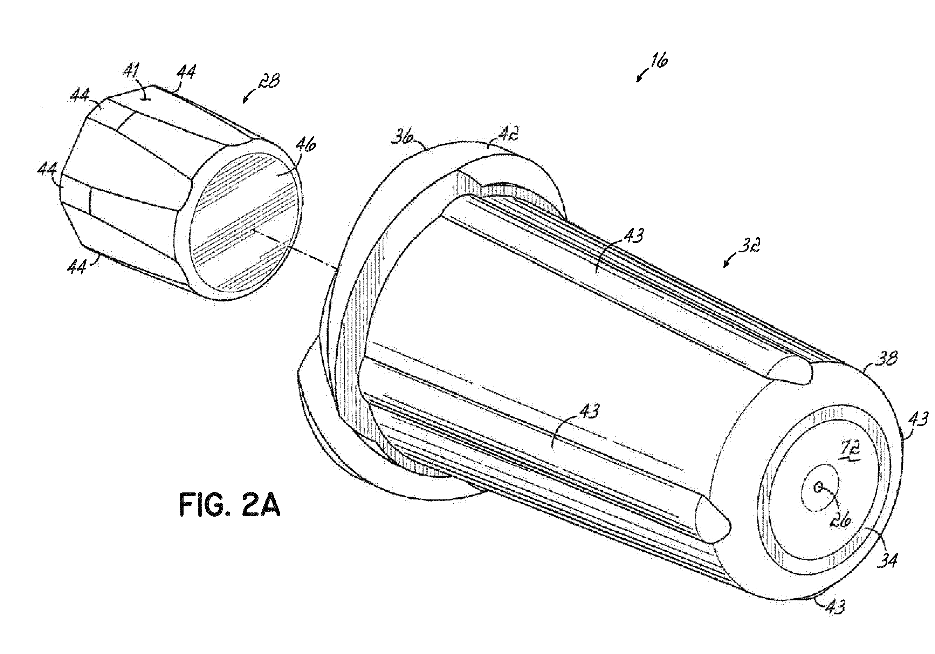

With respect to FIG. 2A and FIG. 2B, the mixing tip 16 generally includes an insert body 28 positioned distally within a tip body 32. The tip body 32 extends along a length to a distal wall 34. At a proximal end 36 of the tip body 32, the tip body 32 defines the inlet 24, whereas, at an opposite distal end 38 of the tip body 32, the distal wall 34 defines the outlet 26. The distal wall 34 inwardly projects from the tip body 32 to define the outlet 26 about a central axis of the tip body 32. In this respect, the distal wall 34 is generally transverse to the length of the tip body 32. The tip body 32 further defines an interior passage 39 extending from the inlet 24 to the outlet 26.

Furthermore, the tip body 32 has an annular flange 42 at the proximal end 36 and a plurality of raised surfaces 43 extending along the length of the tip body 32 from the annular flange 42 to the distal end 38. The annular flange 42 is in the form of a luer connector configured to removably connect to the connector 14. The plurality of raised surfaces 43 are positioned about the tip body 32 for providing enhanced grip during the assembly of the tip body 32 to the connector 14.

The insert body 28 is positioned within the interior passage 39 and against the distal wall 34. The interior passage 39 has a circular inner surface 40 and the insert body 28 has an outer surface 41. Along the outer surface 41 a plurality of ridges 44 extend along a length of the insert body 28. According to an exemplary embodiment, the outer surface 41 has six ridges and is hexagonal. The insert body 28 also includes a distal insert face 46 and an opposing proximal insert face 48. The distal insert face 46 is generally planar and transverse to the length of the tip body 32. In contrast, the proximal insert face 48 includes a blind hole 50 for indicating to a user or manufacturer that the insert body 28 is properly inserted within the interior passage 39 with the distal insert face 46 against the distal wall 34.

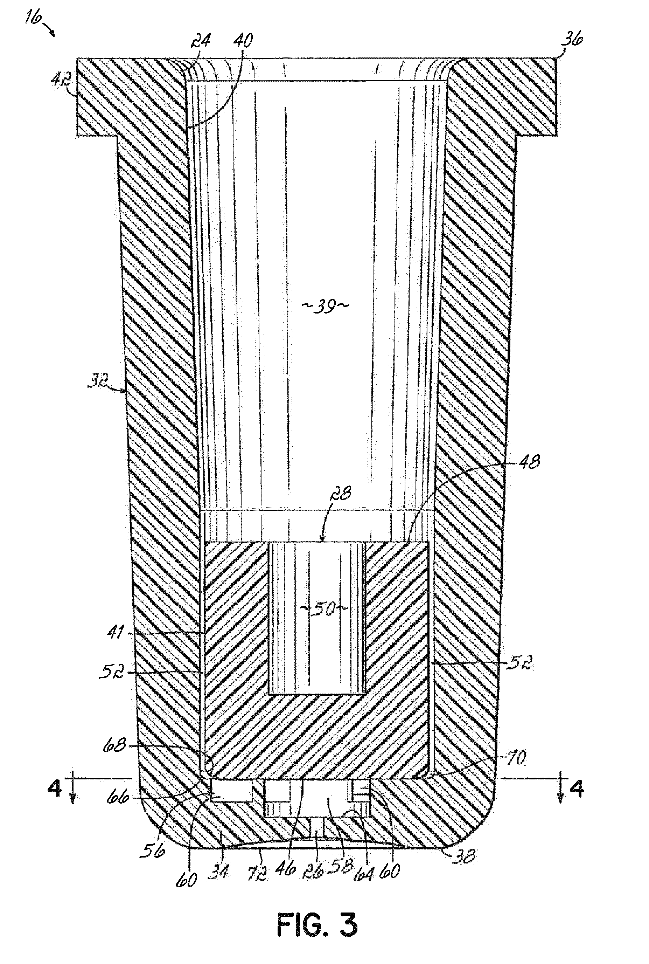

As shown in FIG. 2B and FIG. 3, the insert body 28 and the tip body 32 collectively define a plurality of guide passages 52 extending along the length of the tip body 32 therebetween. More particularly, the plurality of ridges 44 abut against an inner surface 40 of the tip body 32 to center the insert body 28 coaxially within the interior passage 39. In turn, the plurality of ridges 44 are positioned against the inner surface 40 to collectively define each of the guide passages 52. According to an exemplary embodiment, the mixing tip 16 includes six guide passages 52.

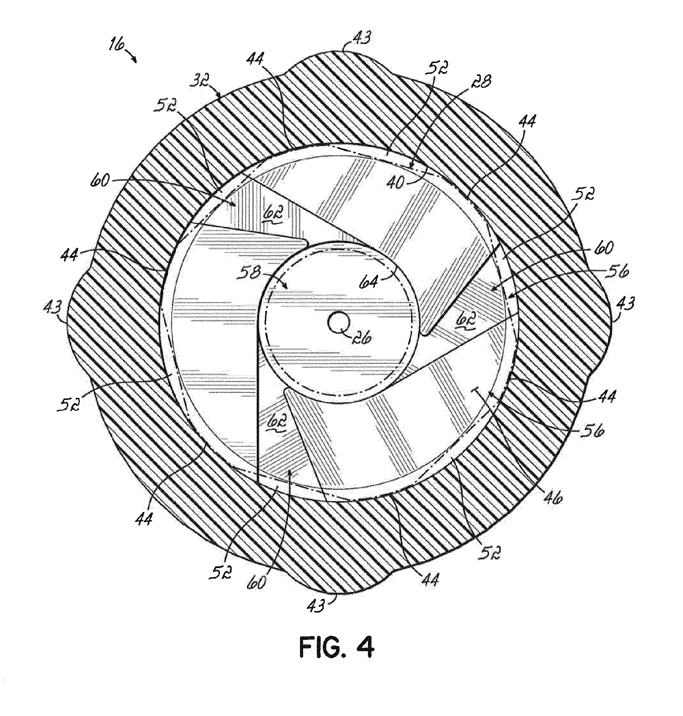

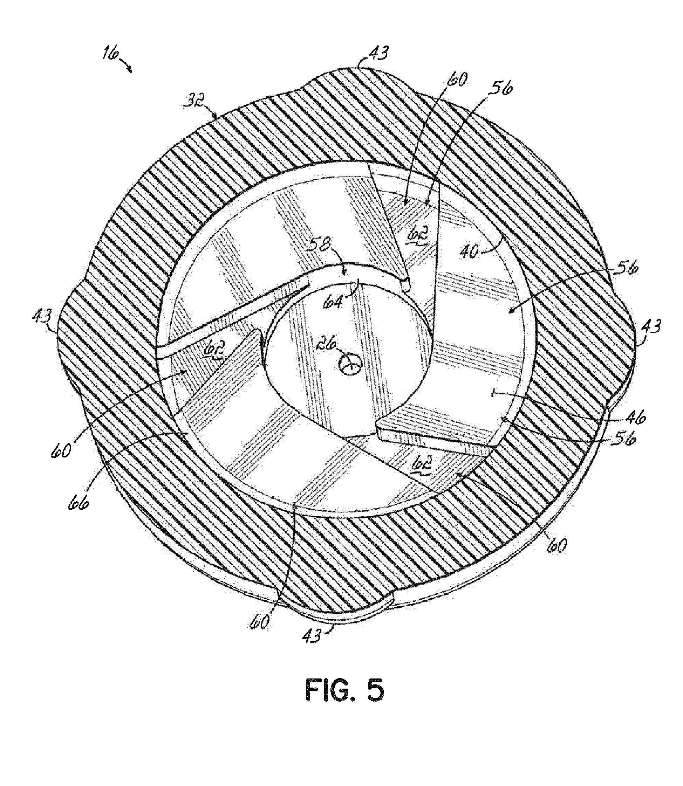

With respect to FIGS. 3-5, the distal wall 34 and the insert body 28 define a static mixing element 56 and a mixing chamber 58 therebetween. The static mixing element 56 includes a plurality of channels 60 between the distal wall 34 and the insert body 28 for directing the first and second fluids into the mixing chamber 58. Each channel 60 is defined by a recess 62 within the distal wall 34 that spirals from the inner surface 40 toward a central bore 64 about the outlet 26. As such, the planar, distal insert face 46 covers each of the recesses 62 and central bore 64 to define the channels 60 and mixing chamber 58, respectively. The plurality of channels 60 thus fluidly connect to the plurality of guide passages 52 for directing the first and second fluids into the mixing chamber 58 and mixing the first and second fluids into a mixed fluid. According to an exemplary embodiment, the static mixing element 56 includes three channels 60.

Advantageously, the insert body 28 and tip body 32 define a number of guide passages 52 that is a multiple of a number of channels 60 with each of the guide passages 52 and channels 60 being radially and evenly distributed within the mixing tip 16 equidistance about the central axis. Accordingly, regardless of the radial orientation in which the insert body 28 is positioned within the interior passage 39, the guide passages 52 will direct relatively balanced flows of the first and second fluids to the channels 60. Thereby, the channels 60 will direct the first and second fluids within the mixing chamber 58 to generate a relatively balanced vortex that mixes the first and second fluids. In addition, the insert body 28 has a relatively large outer radius 66 adjacent to a relatively small inner radius 68 of the inner surface 40 that defines an annular channel 70. The annular channel 70 fluidly connects each of the guide passages 52 at an intersection with the channels 60 to further balance the flows from the guide passages 52.

The mixing chamber 58 extends through the distal wall 34 to the outlet 26. The outlet 26 receives the mixed fluid and is configured to discharge the mixed fluid as a plurality of droplets. According to an exemplary embodiment, the distal wall 34 includes a distal wall face 72, a portion of which is generally convex about the outlet 26.

In use, the applicator 10 shown in FIGS. 1-5 forces the first and second fluids from the first and second syringes 12a, 12b and toward the mixing tip 16. The first and second fluids pass through an inlet 24 and are introduced into the interior passage 39. As the first and second fluids approach the insert body 28, each fluid is directed outward toward the inner surface 40 and into the plurality of guide passages 52 as a first, second, and third flow of fluids. The first, second, and third flow of fluids each increase in speed along the plurality of guide passages 52 toward the distal wall 34. At the distal wall 34, the first second and third flows enter the plurality of channels 60 and are directed inward toward the mixing chamber 58. Each of the first, second, and third flows, moves along first, second, and third channels 60, respectively, and is directed into the mixing chamber 58 to collectively generate a vortex of first and second fluids within the mixing chamber 58. The vortex mixes the first and second fluids into the mixed fluid. The mixed fluid passes through the outlet 26 and discharges from the mixing tip 16. According to an exemplary embodiment, the first and second fluids are blood and thrombin. As such, the mixed blood and thrombin discharge in the form of droplets to be dispensed onto an anatomy of a patient for improved coagulation.

With regard to assembly of the mixing tip 16, the insert body 28 inserts into the tip body 32 to define the plurality of guide passages 52, the plurality of channels 60, and the mixing chamber 58. As such, the annular flange 42 is configured to connect to the connector 14 so as to capture the insert body 28 within the tip body 32 to inhibit the insert body 28 from inadvertently being removed from the tip body 32 during use. According to an exemplary embodiment, the mixing tip 16 is manufactured from a radiopaque plastic to be identifiable via x-rays.

While the present invention has been illustrated by the description of one or more embodiments thereof, and while the embodiments have been described in considerable detail, additional advantages and modifications will readily appear to those skilled in the art. For example, it will be appreciated that the guide passages 52, the channels 60, and mixing chamber 58 may be located wholly or partially on one or both of the insert body 28 and the tip body 32. Thus, while the exemplary embodiment of the mixing tip 16 collectively defines these features, it is not intended to necessarily be limited as such. The various features shown and described herein may be used alone or in any combination.

## Claims

1. A mixing tip for discharging a fluid from a dispensing body for use during a medical procedure, comprising;
a tip body having an interior passage extending along a length, the interior passage including an inlet at a first end configured for fluidly connecting the dispensing body for receiving the fluid from the dispensing body and an outlet in a distal wall at a second end opposite to the first end;
an insert body positioned within the interior passage, a first passage positioned between the insert body and the tip body and fluidly coupling the interior passage with the outlet in the distal wall; and
a mixing chamber positioned between the insert body and the distal wall, the mixing chamber including a static mixing element.

2. The mixing tip of claim 1 wherein the static mixing element includes a first channel, and the first channel extends from the first passage to the mixing chamber for introducing a first flow of the fluid into the mixing chamber.

3. The mixing tip of claim 2 wherein:
the static mixing element includes a second passage and a second channel, the second passage fluidly coupling the interior passage with the outlet in the distal wall for receiving the fluid from the dispensing body, and the second channel extends from the second passage to the mixing chamber for introducing a second flow of the fluid into the mixing chamber.

4. The mixing tip of claim 3 wherein the insert body at least partially defines an annular channel proximate to the distal wall, the annular channel fluidly connecting the first and second channels for balancing pressure therein.

5. The mixing tip of either claim 3 or claim 4 wherein the static mixing element includes a third passage and a third channel, the third passage fluidly coupling the interior passage with the outlet in the distal wall for receiving the fluid from the dispensing body, and the third channel extends from the third passage to the mixing chamber for introducing a third flow of the fluid into the mixing chamber, the first, second, and third channels being radially spaced about the tip body approximately equidistance from each other.

6. The mixing tip of claim 5 wherein the insert body and the tip body have a radial alignment relative to each other, and the mixing tip further comprises a pair of first passages, a pair of second passages, and a pair of third passages, the pair of first, second, and third passages fluidly connected to the inlet for receiving approximately equal portions of the first, second, and third flows from the inlet regardless of the radial alignment between the insert body and the tip body.

7. The mixing tip of claim 6 wherein the tip body has an inner surface and the insert body has an outer surface, the inner surface being circular and the outer surface being hexagonal.

8. The mixing tip of any preceding claim further comprising:
a pair of ridges longitudinally extending along the insert body and further defining the first passage,
wherein the pair of ridges abut the tip body to center the insert body within the interior space coaxially within the inlet.

9. An applicator for dispensing a fluid for use during a medical procedure, comprising a dispensing body for containing the fluid, the dispensing body including a conduit, and a mixing tip as claimed in any preceding claim, the inlet fluidly connected to the conduit for receiving the fluid from the conduit.

10. A method of dispensing a mixed fluid during a medical procedure with a mixing tip, the mixing tip having an insert body positioned within a tip body, comprising introducing a first fluid and a second fluid into an interior passage of the tip body, directing the first and second fluids through a passage between the tip body and the insert body, directing the first and second fluids from the passage into a mixing chamber located between the insert body and a distal wall of the tip body, mixing the first and second fluids within the mixing chamber; and discharging the mixed fluids from the mixing tip.

11. The method of claim 10 further comprising generating a vortex with the first and second fluids to mix the first and second fluid into a mixed fluid.

12. The method of either claim 10 or claim 11 wherein discharging the mixed fluid further includes forming the mixed fluids into a plurality of droplet.

13. The method of any one of claims 10 to 12 wherein the insert body includes at least two ridges further defining the channel, the method further comprising abuting the at least two ridges against an inner surface of the interior passage to center the insert body coaxially within the interior passage.

14. The method of any one of claims 10 to 13, the method further comprising securing the tip body to a dispensing body; and capturing the insert body within the tip body.

15. The method of any one of claims 10 to 14 wherein the first fluid is thrombin and the second fluid is blood, the method further comprising directing the mixed blood and thrombin onto an anatomical site of a patient to promote blood coagulation at the anatomical site.
